# DEMANDE DE BREVET EUROPEEN

(11) **EP 1 437 348 A1**
(43) Date de publication de la demande: **14.07.2004**
(21) Numéro de dépôt: 03293239.4
(22) Date de dépôt: 19.12.2003
(51) Int. Cl.: C07D 239/48, C07F 7/18, C07D 401/12, C07D 405/12, C07D 403/12

(54) **Dérivés de de la 6-méthyl-pyrimidine-2,4-diamine, procédé de synthèse, compositions les comprenant et utilisations comme agents neutralisants basiques**

(30) Priorité: 13.01.2003 FR 0300300; 17.09.2003 FR 0310918
(71) Demandeur: L'OREAL, 75008 Paris (FR)
(72) Inventeur: Breton, Philippe, 78150 Le Chesnay (FR); Dalko, Maria, 91190 Gif s/Yvette (FR); Forestier, Serge, 77410 Claye Souilly (FR); Buiatti, Muriel, 75116 Paris (FR); Lerebour, Géraldine, 78350 Les Loges En Josas (FR)
(74) Mandataire: Dodin, Catherine

(57) **Abrégé**

L'invention se rapporte à de nouveaux dérivés de la famille de la 6-méthyl-pyrimidine-2,4-diamine répondant à la formule (I) suivante : 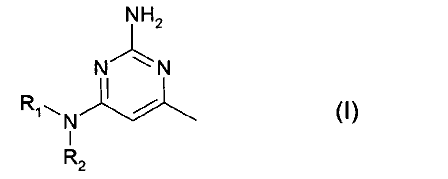 à leur procédé de synthèse, aux compositions cosmétiques ou pharmaceutiques les comprenant, ainsi qu'à leur utilisation comme agents neutralisants basiques ou comme agents conservateurs.

## Description

L'invention se rapporte à de nouveaux dérivés de la famille de la 6-méthyl-2,4-diaminopyrimidine, à leur procédé de synthèse et à leurs utilisations dans des compositions cosmétiques ou pharmaceutiques, notamment dermatologiques, de préférence destinées à une application topique.

Il est courant de chercher à introduire dans les compositions cosmétiques ou dermatologiques, des additifs ou des agents actifs acides qui présentent l'inconvénient de générer des effets secondaires irritants sur la peau et/ou de rendre instable la composition dans laquelle ils sont introduits du fait de cette acidité. Afin de pallier ces inconvénients, il est connu d'introduire dans ces compositions des agents neutralisants basiques organiques ou inorganiques, notamment de l'hydroxyde de sodium ou de la triéthanolamine. Cependant, la triéthanolamine présente une légère odeur ammoniaquée et à une tendance à brunir au contact de l'air et de la lumière, ce qui ne la rend pas toujours compatible dans une composition, notamment cosmétique.
On comprend donc l'importance de pouvoir disposer de nouveaux composés neutralisants basiques, compatibles avec une application topique cutanée et ne présentant pas les inconvénients des dérivés de l'art antérieur.

Par ailleurs, il est usuel d'introduire dans les compositions cosmétiques ou dermatologiques, des conservateurs chimiques destinés à lutter contre le développement des microorganismes au sein de ces compositions, ce qui les rendrait rapidement inaptes à l'utilisation. Il faut notamment protéger les compositions contre les microorganismes susceptibles de se développer à l'intérieur de la composition et également contre ceux que l'utilisateur pourrait y introduire en la manipulant, en particulier lors de la préhension avec les doigts de produits en pot.
Les conservateurs chimiques couramment utilisés sont notamment les parabènes ou les composés libérateurs de formol. Ces conservateurs présentent toutefois l'inconvénient de causer des irritations, en particulier sur les peaux sensibles, lorsqu'ils sont présents à des taux relativement importants. Comme conservateurs connus, on peut également citer les hydroxyacides organiques. Mais ces composés peuvent également générer des irritations du fait de leur effet desquamant sur la peau, ce qui n'est pas toujours bien toléré.
Il subsiste donc le besoin d'agents conservateurs, notamment d'agents anti-microbiens, ayant une action au moins aussi efficace que les composés de l'art antérieur, mais n'en présentant pas les inconvénients. Par ailleurs, il serait souhaitable de disposer d'agents anti-microbiens présentant un spectre anti-microbien au moins aussi large, voire supérieur, à ceux des composés déjà existants.

La demanderesse a constaté de manière surprenante et inattendue, que certains dérivés de la famille de la 6-méthyl-2,4-diaminopyrimidine présentaient de bonnes propriétés anti-microbiennes, que cela soit vis-à-vis des bactéries, des levures ou des moisissures
Par ailleurs, ces dérivés présentent une fonction basique sur le motif pyrimidine dont la valeur de pKa est supérieure ou égal à 7,5, ce qui les rend compatibles en tant qu'agents neutralisants basiques, notamment dans des compositions cosmétiques ou dermatologiques.

Parmi les dérivés de la 6-méthyl-2,4-diaminopyrimidine, on connaît déjà la 2-amino-4-morpholino-6-méthylpyrimidine et la 2-amino-4-pipéridino-6-méthylpyrimidine dont les procédés de synthèse sont décrits par A. Maggiolo et A. Phillips dans J.Am.Chem.Soc., 1950, pp. 376-381. Ce document ne mentionne toutefois aucune utilisation particulière de ces deux composés.
Le document DE19917784 décrit des composés 6-trifluorométhyl-2,4-diamino-pyrimidine comprenant un substituant benzyle ou phényle, et leur utilisation pour la protection phytosanitaire, la protection des matériaux techniques (bois, cuir, carton, textiles, colles à bois) contre les bactéries, les champignons et les moisissures.
Le document DE3717480 décrit des composés 2,6-diamine-pyrimidine halogénés et leur utilisation comme herbicides et microbicides.
Les documents Egypt. J. Pharm. Sci. 1978, 28, (1-4), pp. 117-126 et J. Chem. Soc.1986, pp 914-917, strictement identiques, décrivent notamment la synthèse de composés 6-méthyl-pyrimidine-2,4-diamine dont l'amine en position 2 est substituée par un aryle et rapportent des activités antimicrobiennes pour certains de ces composés.
Toutefois, aucun de ces documents ne mentionne les dérivés selon l'invention, dans les utilisations envisagées selon l'invention.

Un objet de la présente invention est donc une composition cosmétique, dermatologique ou pharmaceutique, comprenant, dans un milieu physiologiquement acceptable, au moins un composé de la famille de la 6-méthyl-pyrimidine-2,4-diamine, de formule (I) telle que définie ci-après.
Un autre objet est l'utilisation d'au moins un tel composé de formule (I) comme agent neutralisant basique.
Un autre objet est l'utilisation d'au moins un tel composé de formule (I) comme agent conservateur, notamment comme agent anti-microbien, comme agent anti-bactérien et/ou comme agent anti-fongique.
Un autre objet de l'invention est un procédé de conservation d'une composition cosmétique, dermatologique ou pharmaceutique, caractérisé en ce qu'il consiste à incorporer dans ladite composition au moins un tel composé de formule (I).
Un autre objet de l'invention est un tel composé de la famille de la 6-méthylpyrimidine-2,4-diamine, de formule (I).
Encore un autre objet de l'invention est un procédé de préparation desdits composés de formule (I).

De par leur spectre anti-microbien large, les dérivés selon l'invention peuvent être employés, notamment dans les compositions cosmétiques, comme agents anti-microbiens, notamment comme agents anti-bactériens, et/ou comme agents anti-fongiques c'est à dire comme agents anti-levures et/ou anti-moisissures. Ils peuvent être employés de manière avantageuse dans des compositions cosmétiques ou pharmaceutiques, notamment dermatologiques, en tant qu'agent conservateur.
Il a en effet été mis en évidence pour les dérivés selon l'invention, des activités anti-microbiennes supérieures par rapport à celles des composés décrits pour leurs activités antimicrobiennes dans l'art antérieur précédemment cité. En particulier, les C.M.I. (Concentration Minimale Inhibitrice) sont meilleures pour les dérivés selon l'invention par rapport auxdits composés l'art antérieur.
En outre, contrairement aux composés de l'art antérieur, les dérivés selon l'invention présentent l'avantage de ne pas comporter de groupements aryles dérivant d'amines aromatiques, notamment des composés de l'aniline et/ou de groupements aryles halogénés, notamment chlorés ou fluorés. Ces substituants sont bien connus pour générer des irritations cutanées et les composés aryles chlorés et/ou fluorés sont connus pour être écotoxiques.
Par ailleurs, les composés selon l'invention présentent comme avantage, une structure chimique clairement définie et caractérisée, d'où une reproductibilité de leur fabrication aisée; leur faisabilité industrielle est également assez simple. De plus, ils possèdent une bonne solubilité ou compatibilité avec les milieux couramment employés en cosmétique, les milieux aqueux notamment.
En outre, ils présentent l'avantage d'être sans odeur.

Les composés selon la présente invention sont donc des dérivés de la famille de la 6-méthyl-2,4-diaminopyrimidine et répondent à la formule (I) suivante : 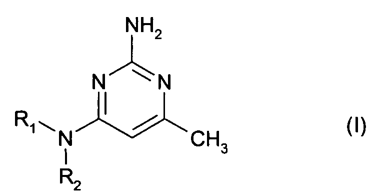 dans laquelle R₁ et R₂ , identiques ou différents, représentent:
- un atome d'hydrogène, étant entendu que R₁ et R₂ ne représentent pas simultanément un atome d'hydrogène,
- un radical hydrocarboné linéaire ou ramifié, saturé ou insaturé, en C1-C20, avantageusement en C1-C6, pouvant être substitué par au moins un radical choisi parmi :
   (i) un radical -NH₂, -CN, -CF₃, -OZ ou -COOZ avec Z représentant un atome d'hydrogène ou un radical alkyl en C1-C4 linéaire ou ramifié, saturé ou insaturé, avantageusement un radical méthyle, éthyle, propyle, isopropyle, butyle, isobutyle ou tertiobutyle;
   (ii) un radical -NRR' avec R et R' identiques ou différents représentent un radical alkyle en C1-C8, avantageusement en C1-C3, linéaire ou ramifié, saturé ou insaturé; R et R' pris ensemble pouvant former avec l'atome d'azote un hétérocycle, qui peut être aromatique, de 5, 6, 7 ou 8 chaînons pouvant comprendre au moins un hétéro-atome additionnel choisi parmi O, N et/ou S, ledit hétérocycle, y compris l'atome d'azote, pouvant être substitué par une chaîne alkyle en C1-C6, ladite chaîne alkyle pouvant être elle-même substituée par au moins un radical -NR"R"' avec R" et R"' représentant indépendamment l'un de l'autre un atome d'hydrogène ou un radical hydrocarboné, saturé ou insaturé, linéaire ou ramifié, en C1-6 ;
   (iii) un radical -Si(CH₃)₂-OH; -Si(CH₃)₂-OEt; -Si(CH₃)₂-O-CH₂-Phényle, -Si(CH₃)₂-OMe ou -Si(iPr)₂-OEt;
   (iv) un hétérocycle de 5, 6, 7 ou 8 chaînons comprenant au moins un hétéroatome choisi parmi O, N et/ou S, ledit hétérocycle, y compris l'azote quand il est présent, pouvant être substitué par une chaîne alkyle en C1-C6, avantageusement en C1-C3 ;
   (v) un hétérocycle aromatique de 5, 6, 7 ou 8 chaînons, comprenant au moins un hétéroatome choisi parmi O, N et/ou S, ledit hétérocycle pouvant être substitué par une chaîne alkyle en C1-C6; avantageusement méthyle, éthyle ou propyle;
   (vi) un cycloalkyle comprenant de 5, 6, 7 ou 8 chaînons, pouvant être substitué par une chaîne alkyle en C1-C6 pouvant elle-même être substituée par au moins un radical -NR"R"' avec R" et R"' représentant indépendamment l'un de l'autre un atome d'hydrogène ou un radical hydrocarboné, saturé ou insaturé, linéaire ou ramifié, en C1-C6; et/ou par un cycloalkyle comprenant de 5, 6, 7 ou 8 chaînons;
- R₁ et R₂ pris ensemble pouvant former avec l'atome d'azote un premier hétérocycle non aromatique de 5, 6, 7 ou 8 chaînons pouvant comprendre au moins un hétéroatome additionnel choisi parmi O, N et/ou S, ledit premier hétérocycle, y compris l'atome d'azote, pouvant être substitué par (a) une chaîne alkyle en C1-C6 pouvant être elle-même substituée par au moins un radical -NR"R"' avec R" et R"' représentant, indépendamment l'un de l'autre, un atome d'hydrogène ou un radical hydrocarboné, saturé ou insaturé, linéaire ou ramifié, en C1-C6; et/ou par (b) un cycloalkyle ou un second hétérocycle, pouvant être aromatique, de 5, 6, 7 ou 8 chaînons, qui peut comprendre au moins un autre hétéroatome choisi parmi O, N et/ou S, ledit cycle ou second hétérocycle pouvant être substitué par une chaîne alkyle en C1-C6;
   ainsi que leurs sels.

Par radical alkyle linéaire ou ramifié en C1-C20, on entend selon l'invention les radicaux acycliques provenant de l'enlèvement d'un atome d'hydrogène dans la molécule d'un hydrocarbure, linéaire ou ramifié ayant de 1 à 20 atomes de carbone et en particulier les radicaux méthyle, éthyle, propyle, isopropyle, butyle, isobutyle, tertiobutyle, pentyle, hexyle, heptyle, octyle ainsi que leurs isomères de position correspondant.

Par hétérocycle, on entend selon l'invention un enchaînement d'atomes fermé sur lui-même et comportant au moins un chaînon (hétéro-atome) différent des autres.

Avantageusement selon l'invention les hétérocycles non aromatiques de 5, 6, 7 ou 8 chaînons qui peuvent comprendre au moins un autre hétéroatome choisi parmi l'oxygène et/ou l'azote et/ou le soufre, sont la pipéridine, la pipérazine, la morpholine, la pyridine, le pyrrole, le pyrazole, l'oxadiazole, le thiadiazole, le triazole, le tétrazole, le thiazole, l'imidazole, la pyrimidine, le furanne, l'indole, le benzimidazole, le benzothioazole, le benzofuranne, le benzothiophène, le benzoxazole.

Selon l'invention, le second hétérocycle, pouvant être aromatique et comprenant de 5, 6, 7 ou 8 chaînons est avantageusement choisi parmi la pipéridine, la pipérazine, la morpholine, la pyridine, le pyrrole, le pyrazole, l'oxadiazole, le thiadiazole, le triazole, le tétrazole, le thiazole, l'imidazole, la pyrimidine, le furanne, l'indole, le benzimidazole, le benzothioazole, le benzofuranne, le benzothiophène, le benzoxazole.

Parmi les sels, notamment d'acide minéral ou d'acide organique, des composés de formule (I), on peut citer les sels obtenus par addition du composé de formule (I) avec un acide minéral choisi parmi les acides chlorhydrique, sulfurique, nitrique et phosphorique, ou avec un acide organique choisi parmi les acides carbonique, acétique, succinique, fumarique, lactique, glycolique, citrique, gluconique, salicylique et tartrique.

De préférence, R1 représente l'hydrogène.

De préférence, R2 est un radical hydrocarboné linéaire ou ramifié, saturé ou insaturé, en C1-C20, avantageusement en C1-C6, pouvant être substitué par
(i) -NH₂,
(iv) un hétérocycle de 5, 6, 7 ou 8 chaînons comprenant au moins un hétéroatome d'azote, ledit hétérocycle, y compris l'azote, pouvant être substitué par une chaîne alkyle en C1-C6, avantageusement en C1-C3 ;
(v) un hétérocycle aromatique de 5, 6, 7 ou 8 chaînons, comprenant au moins un hétéroatome d'oxygène;
(vi) un cycloalkyle comprenant de 5, 6, 7 ou 8 chaînons, pouvant être substitué par une chaîne alkyle en C1-C6 pouvant elle-même être substituée par au moins un radical -NH2.

De préférence également, R₁ et R₂ pris ensemble forment avec l'atome d'azote un premier hétérocycle non aromatique de 5, 6, 7 ou 8 chaînons pouvant comprendre au moins un hétéroatome additionnel d'azote, ledit premier hétérocycle, y compris l'atome d'azote, pouvant être substitué par (a) une chaîne alkyle en C1-C6 pouvant être elle-même substituée par au moins un radical -NR"R"' avec R" et R"' représentant, indépendamment l'un de l'autre, un radical hydrocarboné, saturé ou insaturé, linéaire ou ramifié, en C1-C6; et/ou par (b) un cycloalkyle ou un second hétérocycle, pouvant être aromatique, de 5, 6, 7 ou 8 chaînons, qui peut comprendre au moins un autre hétéroatome choisi parmi O, ledit cycle ou second hétérocycle pouvant être substitué par une chaîne alkyle en C1-C6.

Parmi les composés répondant à la formule (I), on préfère tout particulièrement les composés suivants :
- 4-N-[4-(methoxydimethylsilyl)butyl]-6-methyl-pyrimidine-2,4-diamine;
- 4-N-(4-(n-butyl-dimethyl-silanol)) 6-methyl-pyrimidine-2,4-diamine;
- 4-N-[-[3-(ethoxydimethylsilyl)propyl]-6-methyl-pyrimidine-2,4-diamine;
- 4-N-[-[3-(ethoxydiisopropylsiIyl)propyl]-6-methyl-pyrimidine-2,4-diamine;
- 4-N-[4-piperidinylmethyl]-6-methyl-pyrimidine-2,4-diamine;
- 4-N-[(2S)-2-pyrrolidinylmethyl]-6-methyl-pyrimidine-2,4-diamine;
- 4-N-[-[1-ethylpyrrolidinyl-2-ylmethyl]-6-methyl-pyrimidine-2,4-diamine;
- 4-methyl-6-[4-(3-pyrrolidin-1-yl-propyl)-[1,4]diazepan-1-yl]-pyrimidin-2-ylamine
- 4-Methyl-6-(4-methyl-[1,4]diazepan-1-yl)-pyrimidin-2-ylamine
- 2-[4-(2-Amino-6-methyl-pyrimidin-4-yl)-[1,4]diazepan-1-yl]-éthanol ;
- 4-(4-Butyl-[1,4]diazepan-1-yl)-6-methyl-pyrimidin-2-ylamine ;
- 4-Methyl-6-(4-phenethyl-[1,4]diazepan-1-yl)-pyrimidin-2-ylamine;
- 4-{4-[2-(4-Fluoro-phenyl)-ethyl]-[1,4]diazepan-1-yl}-6-methyl-pyrimidin-2-ylamine
- 4-Methyl-6-[4-(3-trifluoromethyl-pyridin-2-yl)-[1,4]diazepan-1-yl]-pyrimidin-2-ylamine
- 4-[4-(2-dimethylamino-ethyl)-piperazin-1-yl]-6-methyl-pyrimidin-2-ylamine ;
- 4-Methyl-6-(4-methyl-piperazin-1-yl)-pyrimidin-2-ylamine ;
- 2-[4-(2-Amino-6-methyl-pyrimidin-4-yl)-piperazin-1-yl]-éthanol ;
- 3-[4-(2-Amino-6-methyl-pyrimidin-4-yl)-piperazin-1-yl]-propionitrile ;
- 4-[4-(2-Methoxy-ethyl)-piperazin-1-yl]-6-methyl-pyrimidin-2-ylamine ;
- 4-(4-Cylopentyl-piperazin-1-yl)-6-methyl-pyrimidin-2-ylamine ;
- 4-[4-(3-Methoxy-propyl)-piperazin-1-yl]-6-methyl-pyrimidin-2-ylamine ;
- 4-Methyl-6-(4-phenyl-piperazin-1-yl)-pyrimidin-2-ylamine ;
- 4-Methyl-6-(4-pyridin-2-yl-piperazin-1-yl)-pyrimidin-2-ylamine ;
- 4-Methyl-6-(4-pyrimidin-2-yl-piperazin-1-yl)-pyrimidin-2-ylamine ;
- 4-Methyl-6-(2,3,5,6-tetrahydro-[1,2']bipyrazinyl-4-yl)-pyrimidin-2-ylamine ;
- 4-(4-Cyclohexyl-piperazin-1-yl)-6-methyl-pyrimidin-2-ylamine ;
- 4-(4-Hexyl-piperazin-1-yl)-6-methyl-pyrimidin-2-ylamine ;
- 4-N-[-(2-amino-2-methyl-propyl)-6-methyl-pyrimidine-2,4-diamine ;
- 4-N-[-(2-piperazin-1-yl-ethyl)-6-methyl-pyrimidine-2,4-diamine ;
- 4-N-[-(5-methyl-furan-2-ylmethyl)-6-methyl-pyrimidine-2,4-diamine ;
- 4-N-[(Furan-2-ylmethyl-)6-methyl-pyrimidine-2,4-diamine ;
- 4-Methyl-6-[4-(1-methyl-piperidin-4-yl)-piperazin-1-yl]-pyrimidin-2-ylamine ;
- 4-Methyl-6-(4-imidazol-2-yl-piperazin-1-yl)-pyrimidin-2-ylamine ;
- 4-N-[-(3-aminomethyl-cyclohexylmethyl)-6-methyl-pyrimidine-2,4-diamine ;
- 4 Methyl-6-pipéridine-1-yl-pirimidine-2-ylamine ;
- 4-Methyl-6-pyrrolidin-1-yl-pyrimidin-2-ylamine ;
- 1-(2-Amino-6-methyl-pyrimidin-4-yl)-pyrrolidin-3-ol ;
- [1-(2-Amino-6-methyl-pyrimidin-4-yl)-pyrrolidin-3-yl]-méthanol ;
- 4-Methyl-6-(2-methyl-pyrrolidin-1-yl)-pyrimidin-2-ylamine ;
- 4-(2-Isopropyl-pyrrolidin-1-yl)-6-methyl-pyrimidin-2-ylamine ;
- 1-(2-Amino-6-methyl-pyrimidin-4-yl)-pyrrolidine-2-carbonitrile ;
- 4-Methyl-6-(3-phenyl-pyrrolidin-1-yl)-pyrimidin-2-ylamine ;
- 4-Methyl-6-(3-pyridin-3-yl-pyrrolidin-1-yl)-pyrimidin-2-ylamine ;
- 4-Methyl-6-(3-pyridin-2-yl-pyrrolidin-1-yl)-pyrimidin-2-ylamine ;
- 4-N-Hexyl-6-methyl-pyrimidine-2,4-diamine.

Les composés de formule (I) sont des composés nouveaux, à l'exception de la 2-amino-4-morpholino-6-méthylpyridine et de la 2-amino-4-pipéridino-6-méthylpyridine, qui sont décrits dans l'art antérieur cité ci-dessus (*J.Am. Chem. Soc. ,* 1950, 376-381).

Les composés de formule (I) selon l'invention sont facilement accessibles du point de vue synthèse : ils peuvent être obtenus en une seule étape à partir de la 2-amino-4-chloro-6-methyl-pyrimidine ou de la 2-amino-4-methoxy-6-methyl-pyrimidine qui sont des produits commerciaux et d'une base, notamment une amine primaire ou secondaire.
La formation des composés de formule (I) résulte de la substitution nucléophile aromatique d'un chlore ou d'un méthoxy, provenant de la 2-amino-4-chloro-6-methyl-pyrimidine ou de la 2-amino-4-methoxy-6-methyl-pyrimidine, par une amine primaire ou secondaire. Ce type de réaction est amplement documenté. On pourra se référer notamment à l'un des documents suivants :
- Quintela et al., *Monatsh Chem* 1996, 127 (6), 739-745 ;
- Abdel-Fattah et al. ; *J Chem Res, Synop* 1994 (11), 412-413 ;
- Hull et al.; *J Chem Soc* 1946, 357 ;
- Curd et al ; *J Chem Soc* 1946, p 343 et p 362.

Avantageusement, le procédé de préparation des composés de formule (I) est caractérisé en ce qu'il consiste essentiellement en les étapes suivantes:
(i) dissoudre une amine primaire ou secondaire dans un solvant organique polaire, de préférence choisi parmi l'éthanol, méthanol, THF, dioxanne et N-methyl-pyrrolidone;
(ii) ajouter une base organique ou inorganique, de préférence choisie parmi le carbonate de potassium (K₂CO₃), le carbonate de sodium (Na₂CO₃), le carbonate de calcium (CaCO₃), la soude, la diisopropylethylamine, de préférence la diisopropylethylamine, en une quantité comprise entre 1 et 3 équivalents, préférentiellement entre 1 et 2 équivalents, avantageusement 1,5 à 1,7 équivalents ;
(iii) ajouter la 2-amino-4-chloro-6-methyl-pyrimidine ou la 2-amino-4-methoxy-6-methyl-pyrimidine, de préférence la 2-amino-4-chloro-6-methyl-pyrimidine, en une quantité comprise 0,5 et 1,1 équivalents, avantageusement 0,7 à 0,9 équivalents ;
(iv) laisser le milieu à température ambiante ou le porter à une température comprise entre 20°C et 85°C, de préférence à reflux du solvant, pendant une durée comprise entre 2h et 48h, avantageusement entre 12h et 30h, de préférence 20- 26 h ;
(v) éventuellement évaporer sous vide à sec le milieu réactionnel après retour à température ambiante ;
(vi) éventuellement dissoudre le résidu dans de l'eau et extraire la solution aqueuse avec un solvant organique choisi de préférence parmi l'acétate d'éthyle, le dichlorométhane, le diéthyléther, de préférence l'acétate d'éthyle ;
(vii) éventuellement sécher et évaporer à sec la phase organique puis éventuellement purifier le résidu, notamment par chromatographie sur colonne de gel de silice.

Les composés de formule (I) sont, pour certains d'entre eux, nouveaux. Parmi ces composés nouveaux, on préfère tout particulièrement ceux pour lesquels:
- R1 représente l'hydrogène; et/ou
- R2 est un radical hydrocarboné linéaire ou ramifié, saturé ou insaturé, en C1-C20, avantageusement en C1-C6, de préférence substitué par :
   (i) -NH₂,
   (iv) un hétérocycle de 5, 6, 7 ou 8 chaînons comprenant au moins un hétéroatome d'azote, ledit hétérocycle, y compris l'azote, pouvant être substitué par une chaîne alkyle en C1-C6, avantageusement en C1-C3 ;
   (v) un hétérocycle aromatique de 5, 6, 7 ou 8 chaînons, comprenant au moins un hétéroatome d'oxygène;
   (vi) un cycloalkyle comprenant de 5, 6, 7 ou 8 chaînons, pouvant être substitué par une chaîne alkyle en C1-C6 pouvant elle-même être substituée par au moins un radical -NH2; et/ou
      - R₁ et R₂ pris ensemble forment avec l'atome d'azote un premier hétérocycle non aromatique de 5, 6, 7 ou 8 chaînons pouvant comprendre au moins un hétéroatome additionnel d'azote, ledit premier hétérocycle, y compris l'atome d'azote, pouvant être substitué par (a) une chaîne alkyle en C1-C6 pouvant être elle-même substituée par au moins un radical -NR"R"' avec R" et R"' représentant, indépendamment l'un de l'autre, un radical hydrocarboné, saturé ou insaturé, linéaire ou ramifié, en C1-C6; et/ou par (b) un cycloalkyle ou un second hétérocycle, pouvant être aromatique, de 5, 6, 7 ou 8 chaînons, qui peut comprendre au moins un autre hétéroatome choisi parmi O, ledit cycle ou second hétérocycle pouvant être substitué par une chaîne alkyle en C1-C6.

Les composés de formule (I) suivant l'invention peuvent être employés de manière avantageuse dans des compositions cosmétiques ou pharmaceutiques, notamment dermatologiques, comprenant par ailleurs un milieu physiologiquement acceptable, c'est-à-dire compatible avec la peau, ses phanères (cils, ongles, cheveux), les muqueuses et/ou le cuir chevelu, et notamment un milieu cosmétiquement ou pharmaceutiquement acceptable.

Les composés de formule (I) peuvent être présents dans les compositions cosmétiques ou pharmaceutiques en une quantité suffisante pour obtenir l'effet recherché, et qui peut être notamment de 0,001 à 30% en poids, de préférence de 0,01 à 15% en poids, et plus particulièrement de 0,1 à 5% en poids, par rapport au poids total de la composition.

Les compositions comprenant lesdits composés peuvent se présenter sous toutes les formes galéniques appropriées pour une application topique, notamment sous forme de solution aqueuse, hydroalcoolique, organique ou huileuse; de suspension ou de dispersion dans des solvants ou des corps gras, de type lotion ou sérum; sous forme de dispersion vésiculaire; sous forme d'émulsion E/H, H/E ou multiple telle qu'une crème ou un lait; sous forme de pommade, de gel, de bâtonnet solide, de produits anhydres pâteux ou solides, de mousse notamment aérosol ou de spray.

Le milieu physiologiquement acceptable, ses constituants, leur quantité, la forme galénique de la composition et son mode de préparation, peuvent être choisis par l'homme du métier sur la base de ses connaissances générales en fonction du type de composition recherchée.

Notamment, la composition peut comprendre tout corps gras usuellement utilisé dans le domaine d'application envisagé. On peut notamment citer les corps gras siliconés tels que les huiles, les gommes et les cires de silicone, ainsi que les corps gras non siliconés tels que les huiles et les cires d'origine végétale, minérale, animale et/ou synthétique. Les huiles peuvent éventuellement être volatiles ou non volatiles. On peut encore citer les hydrocarbures, les esters et les éthers de synthèse, les alcools gras et les acides gras.
La composition peut également comprendre un milieu aqueux qui comprend de l'eau; un milieu hydroalcoolique comprenant un monoalcool notamment en C1-C6 tel que l'éthanol ou l'isopropanol, ou un milieu organique comprenant des solvants organiques usuels tels que des monoalcools en C₁₋₆, notamment l'éthanol et l'isopropanol, des glycols tels que le propylène glycol, des cétones.
La composition peut comprendre au moins un émulsionnant classique, choisi parmi les émulsionnants amphotères, anioniques, cationiques ou non ioniques, utilisés seuls ou en mélange.
Elle peut également comprendre les adjuvants habituels dans le domaine considéré, tels que les épaississants ou gélifiants hydrophiles ou lipophiles, les additifs hydrophiles ou lipophiles, les actifs notamment cosmétiques, les antioxydants, les parfums, les charges, les pigments, les filtres UV, les absorbeurs d'odeur, les colorants, les hydratants (glycérine), des vitamines, des acides gras essentiels, des polymères liposolubles notamment hydrocarbonés, les opacifiants, les stabilisants, les séquestrants, les conditionneurs, les agents propulseurs.
Bien entendu l'homme du métier veillera à choisir ce ou ces éventuels adjuvants complémentaires et/ou leur quantité de manière telle que les propriétés avantageuses de la composition selon l'invention ne soient pas, ou substantiellement pas, altérées par l'adjonction envisagée.

Avantageusement, la composition selon l'invention comprend au moins un actif additionnel. Comme actifs, on peut citer les composés choisis parmi, seuls ou en mélange, les agents desquamants; les agents hydratants; les agents dépigmentants ou propigmentants; les agents anti-glycation; les inhibiteurs de NO-synthase; les agents stimulant la synthèse de macromolécules dermiques ou épidermiques et/ou empêchant leur dégradation; les agents stimulant la prolifération des fibroblastes et/ou des kératinocytes ou stimulant la différenciation des kératinocytes; les agents myorelaxants; les agents tenseurs; les agents anti-pollution et/ou anti-radicalaire; les agents agissant sur la microcirculation; les agents agissant sur le métabolisme énergétique des cellules.

De préférence, la composition comprend au moins un additif acide et/ou un actif à effet secondaire irritant, de préférence choisi parmi les acides alpha- et bêta-hydroxy-carboxyliques ou alpha et bêta-cétocarboxyliques, et plus particulièrement les alpha- et bêta-hydroxyacides tels que l'acide citrique, l'acide malique, l'acide mandélique, l'acide tartrique, l'acide lactique, l'acide glycolique, de manière générale les acides de fruits, l'acide salicylique et l'acide n-octanoyl-5-salicylique et l'acide ascorbique.

Le pH des compositions selon l'invention est de préférence compris entre 5 et 9, de préférence entre 6 et 8,5, avantageusement de 7.

Les composés de formule (I) peuvent notamment être employés en tant qu'agents neutralisants basiques.
Ils présentent en outre l'avantage de pouvoir être formulés dans des compositions ayant une bonne stabilité, sans qu'il soit nécessaire d'ajouter un agent conservateur. Par agent conservateur, on entend une substance qui est ajoutée communément à une composition afin d'assurer sa conservation vis-à-vis d'un agent contaminant. Avantageusement, Il s'agit des substances décrites comme agent conservateur dans le dictionnaire CTFA et celles figurant à l'annexe VI de la Directive cosmétique européenne.
De plus, les composés de formule (1) suivant l'invention peuvent être avantageusement utilisés comme agent antimicrobien, notamment comme agent anti-bactérien et/ou comme agent antifongique. En effet, on a constaté qu'ils présentent une activité anti-microbienne, notamment une activité anti-bactérienne et anti-fongique, remarquable. Selon la nature des substituants R₁ et/ou R₂ il est possible d'obtenir des composés plus ou moins actifs vis-à-vis de tel type de microbe. On peut ainsi moduler le champ d'action des composés selon l'invention, en focalisant sur tel microbe, en choisissant de manière adéquate la nature chimique du composé employé.

Les compositions comprenant les composés de formule (I) peuvent se présenter :
- sous la forme d'un produit de maquillage de la peau du visage, du corps ou des lèvres, tel qu'un fond de teint, un fard à joues ou à paupières, un stick anti-cernes, un stick camouflant, un eye-liner, un mascara, un rouge à lèvres, un vernis-à-ongles, un soin des ongles;
- sous la forme d'un produit de soin cosmétique de la peau du visage, du corps y compris le cuir chevelu, des lèvres, tel qu'une base de soin pour les lèvres, une base fixante à appliquer sur un rouge à lèvres classique, une composition solaire ou de bronzage artificiel; une composition de soin (de jour, de nuit, antirides, hydratante, etc.) pour le visage; un gel ou une crème de nettoyage ou de démaquillage;
- sous la forme d'une composition pharmaceutique, notamment dermatologique;
- sous la forme d'une composition solide telle qu'un savon;
- sous la forme d'une composition pour aérosol comprenant notamment un agent propulseur sous pression;
- sous la forme d'une composition capillaire, et notamment un shampooing, une lotion de mise en plis, une lotion traitante, une crème ou un gel coiffant, une composition de teinture (notamment d'oxydation) éventuellement sous forme de shampooing colorant, de lotion restructurante pour cheveux, une composition de permanente,
- sous la forme d'une composition à usage bucco-dentaire, par exemple une pâte dentifrice.

Les exemples qui suivent illustrent l'invention plus en détail sans en limiter en aucune façon la portée.

### Exemple 1

Synthèse du 4-(2-Amino-6-methyl-pyrimidin-4-ylamino)-butyl-dimethyl-silanol

Dans un ballon monocol, on introduit le 4-aminobutyl dimethylméthoxysilane (4.45g, 27.6 mmol), le 2-amino-4-chloro-6-methyl-pyrimidine (3.30g, 23 mmol) et la diisopropylethylamine (8 mL, 46 mmol) dans 150 ml d'éthanol. Le milieu est chauffé au reflux pendant 24 heures, puis refroidi et le solvant est évaporé. L'huile obtenue est purifiée par chromatographie sur gel de silice en éluant par dichlorométhane / méthanol 9/1 (v/v).

RMN (¹H, 400MHz, δ ppm, DMSO-d₆) : 6.69 (s, 1H); 5.84 (s, 2H); 5.59 (s, 1H); 3.16 (d, 2H); 2.00 (s, 3H); 1.49 (tt, 2H); 1.34 (tt, 2H); 0.48 (m, 2H); 0.01 (s, 6H) MS (ES +) : 255 (M+H⁺) 100%

### Exemple 2

Les composés de formule (I) suivant l'invention peuvent également être synthétisés suivant le mode opératoire général suivant:
Dans un tube, on introduit la 2-amino-4-chloro-6-methyl-pyrimidine (28.7 mg, 0.2 mmole), la diisopropylethylamine (70 µL, 0.4 mmol) et l'amine correspondante (0.24 mmole). Le milieu est chauffé au reflux pendant 24 heures. Le milieu réactionnel est concentré puis purifié par chromatographie sur colonne de gel de silice. Les produits sont détectés en spectrométrie de masse en ionisation par electrospray.

La 4-N-hexyl-6-methyl-pyrimidine-2,4-diamine a été synthétisée suivant ce mode opératoire général.
RMN (¹H, 400MHz, δ ppm, DMSO-d₆) : 7.74 (s, 1H) ; 6.71 (s, 2H) ; 5.74 (s, 1H) ; 2.08 (s, 3H) ; 1.47 (m, 3H) ; 1.26 (m, 7H) ; 0.86 (m, 3H).

### Exemple 3

Détermination des constantes d'ionisation des composés de formule (I)

### Matériel et méthode :

Les constantes d'ionisation sont déterminées expérimentalement par titrage potentiométrique suivant les conditions opératoires décrites ci-dessous et évaluées par le logiciel de prédiction ACD/pK DB, Version 5.12.

Par défaut, le logiciel utilise une base de données interne pour calculer la valeur de pKa d'une fonction ionisable. La précision du résultat est plus grande en utilisant une base de données qui contient des sous-structures avec une valeur de pKa déterminée expérimentalement.

On utilise une base contenant les 2 molécules suivantes (pKa expérimental dans l'eau, NaCl 0,15M):
- 4-aminopyrimidine : pKa = 6.00
- 2-aminopyrimidine : pKa = 3.90

Conditions opératoires du titrage potentiométrique
- Matériel utilisé : (Radiometer-Tacussel) :
   titrateur TIM 900
   passeur d'échantillons SAC 90
   logiciel Tim Talk 9
   - Prise d'essai : 10⁻⁴ mole
   - Mise en solution : (solubilisation avec passeur d'échantillons)
      - 5 ml du mélange DMSO 70 / Ethanol 10 / Eau 20 (agitation 3 minutes avec barbotage d'azote)
      - 10 ml d'une solution de NaCl 0.225M dans eau 80 / DMSO 20 (agitation 3 minutes avec barbotage d'azote)
   - Méthode de titrage :
      Etalonnage de l'électrode entre pH4 et pH10
      Titrage direct par NaOH 1N ou après ajout d'HCl 1N
      Détection d'inflexion avec ajout continu du titrant
      vitesse d'addition mini : 1%/min
      vitesse d'addition maxi : 1%/min
      Flux d'azote à la surface de la solution pendant le titrage
   - Calculs :
      2 fonctions polynomiales sont utilisées pour obtenir le pKa dans l'eau en milieu NaCl 0.15M :
      pour les fonctions acides : pKa_{H2O} = -0,0208(pKaₛₒₗᵥₐₙₜ)² + 1,257pKaₛₒₗᵥₐₙₜ - 1,6
      pour les fonctions basiques :pKa_{H2O} = -0,0208(pKaₛₒₗᵥₐₙₜ)² + 1,257pKaₛₒₗᵥₐₙₜ - 0,75

### Résultats

La structure des molécules et les valeurs de pKa obtenues par voie expérimentale et par prédiction sont présentées dans les tableaux ci dessus.

La valeur du pKa de la fonction NH⁺/N du motif pyrimidine est indiquée en ca ractère gras.

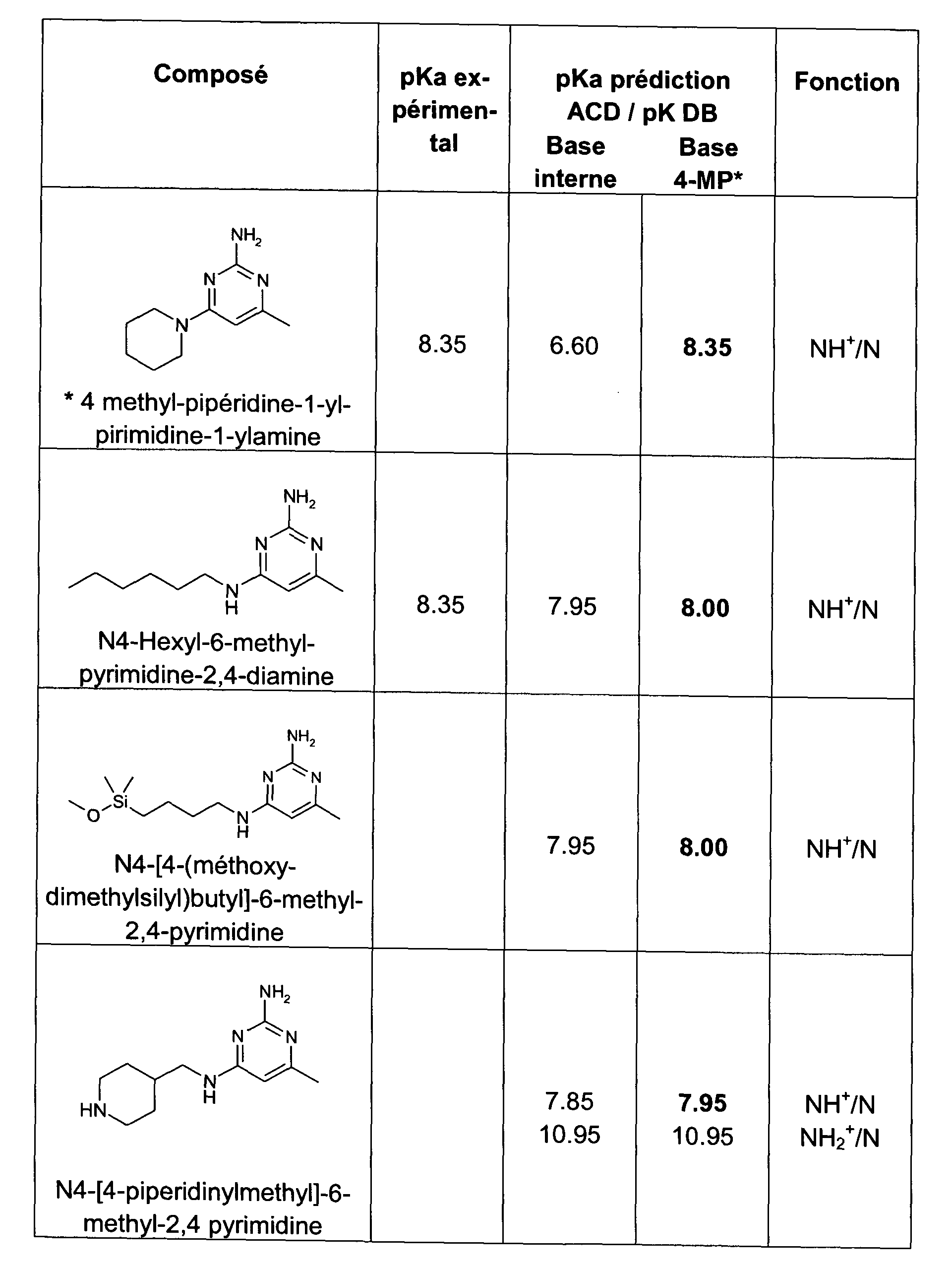

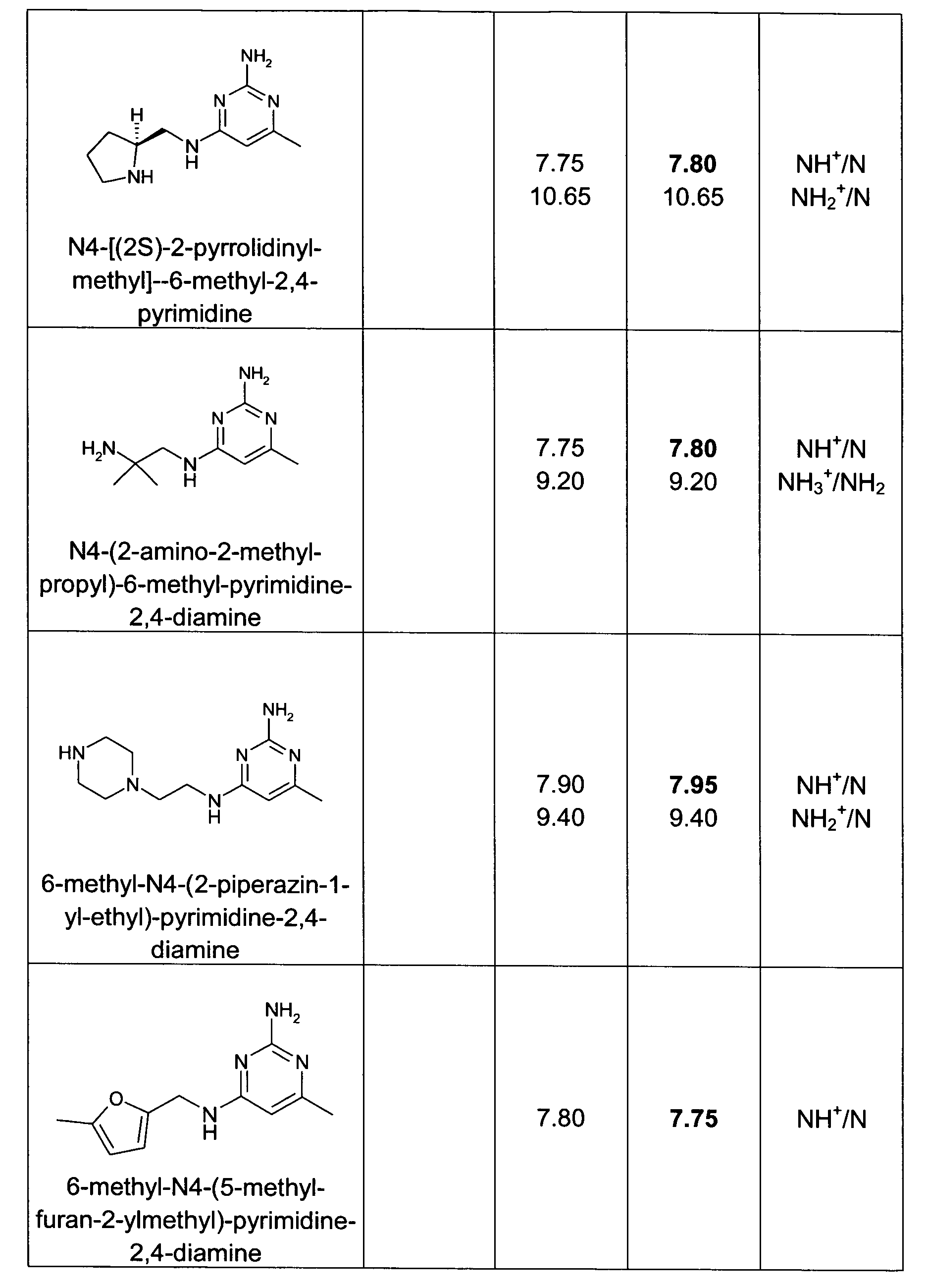

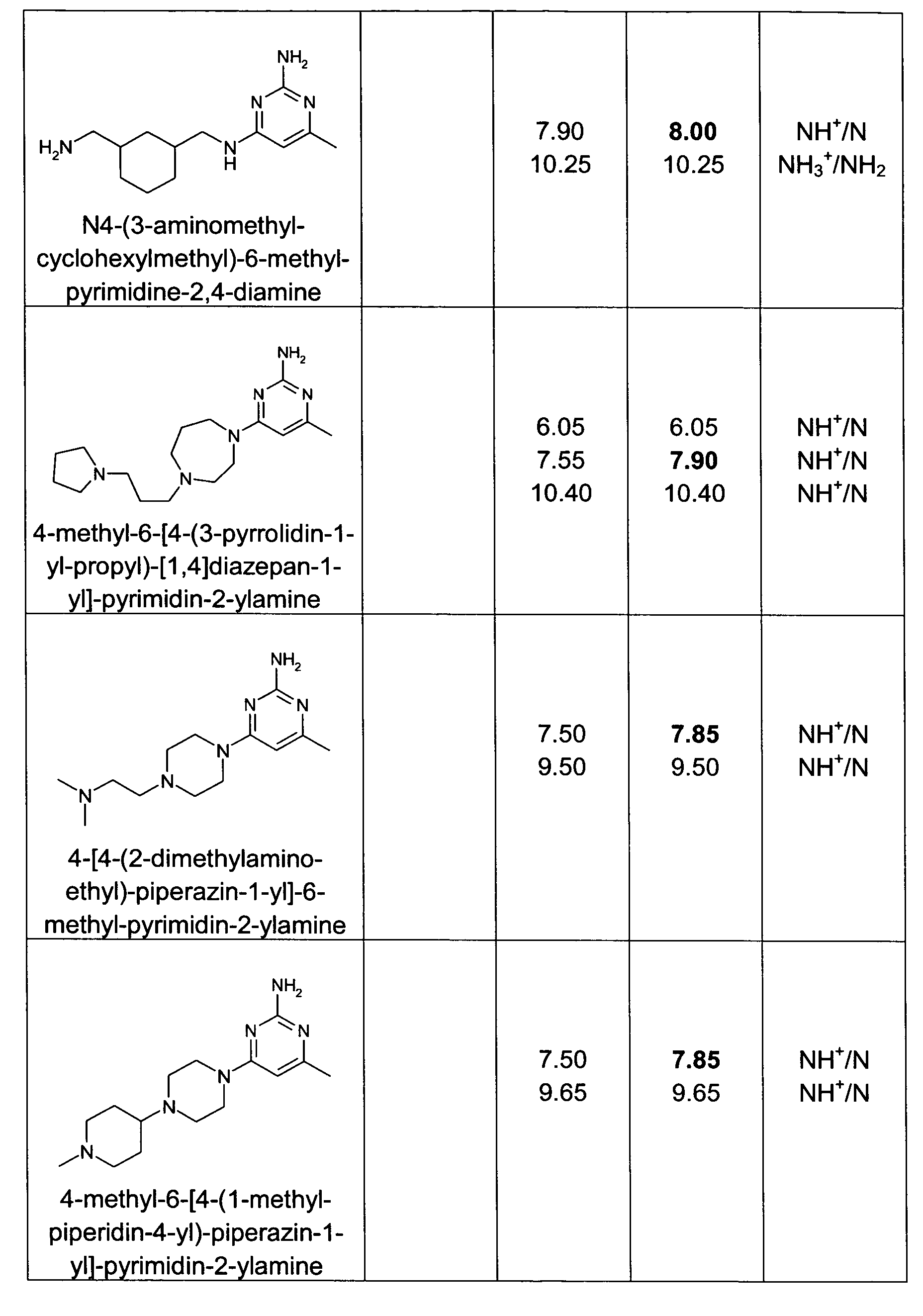

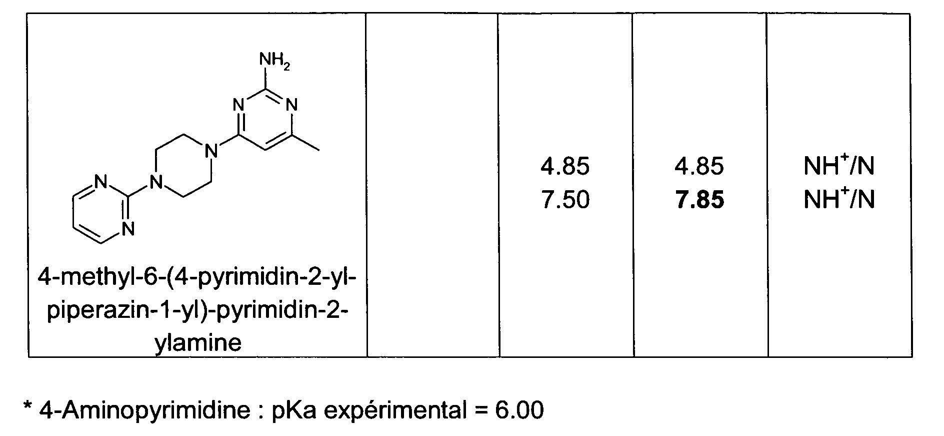

Les composés selon l'invention présentent une fonction basique sur le motif pyrimidine dont la valeur de pKa est supérieure ou égal à 7,5, ce qui les rend compatible en tant qu'agents neutralisants basiques.

### Exemple 4

Etude de détermination de l'activité anti-microbienne de composés selon l'invention

La méthode de détermination de l'activité anti-microbienne des composés selon l'invention fait intervenir la mesure de la Concentration Minimale Inhibitrice (CMI) vis-à-vis de 2 bactéries gram- (E. *coli* et *P. aeruginosa),* d'une bactérie gram+ (S. *aureus*) et d'une levure (*C. albicans*).
Cette méthode a été effectuée selon la méthode décrite dans les articles suivants:
- Baker C.N., Banerjee S.N. and Tenover F.C. : Evaluation of Alamar colorimetric MIC Method for antimicrobial susceptibility testing of gram-negative bacteria. J. Clin. Microbiol. (1994), 32, 1261-1267;
- Baker C.N. and Tenover F.C. : Evaluation of Alamar colorimetric broth microdilution susceptibility testing method for staphylococci and enterococci. J. Clin. Microbiol. (1996), 34, 2654-2659.
Les souches bactériennes utilisées au départ sous forme de disques lyophilisés calibrés sont réhydratées en suivant les indications du fournisseur (Fisher Scientific S.A.)

| Souches | Référence ATCC (American Type Culture Collection) |
|---|---|
| *Escherichia coli* | ATCC #8739 |
| *Staphylococcus aureus* | ATCC #6538 |
| *Pseudomonas aeruginosa* | ATCC #9027 |
| *Candida albicans* | ATCC #10231 |

Les souches sont ensemencées dans du milieu Trypto-caseine-soja. La mise en contact du composé à tester solubilisé dans l'eau, avec les souches en phase de croissance s'effectue en milieu liquide.
Après 16 heures de contact du composé avec les bactéries (37°C), de l'Alamar Blue est ajouté comme indicateur métabolique de la viabilité de cellules variées et de bactéries. La réduction de l'indication est proportionnelle à l'activité métabolique des bactéries.
A l'issue de l'incubation d'une durée de 2h (sauf pour *C. albicans* pour laquelle l'incubation est de 4h), l'activité métabolique des bactéries est évaluée par une lecture en absorbance ou en fluorescence. En fluorescence, les lectures sont réalisées à 544nm (Excitation) et à 590nm (Emission).
Les composés les plus actifs sont sélectionnés et un effet dose (8 concentrations) est ensuite réalisé comme précédemment.
La Concentration Minimale Inhibitrice (C.M.I.) a alors été déterminée comme étant la concentration la plus faible du composé testé pour laquelle était observée une inhibition de croissance de la bactérie ou de la levure ≥ 90%.

On obtient les résultats suivants :

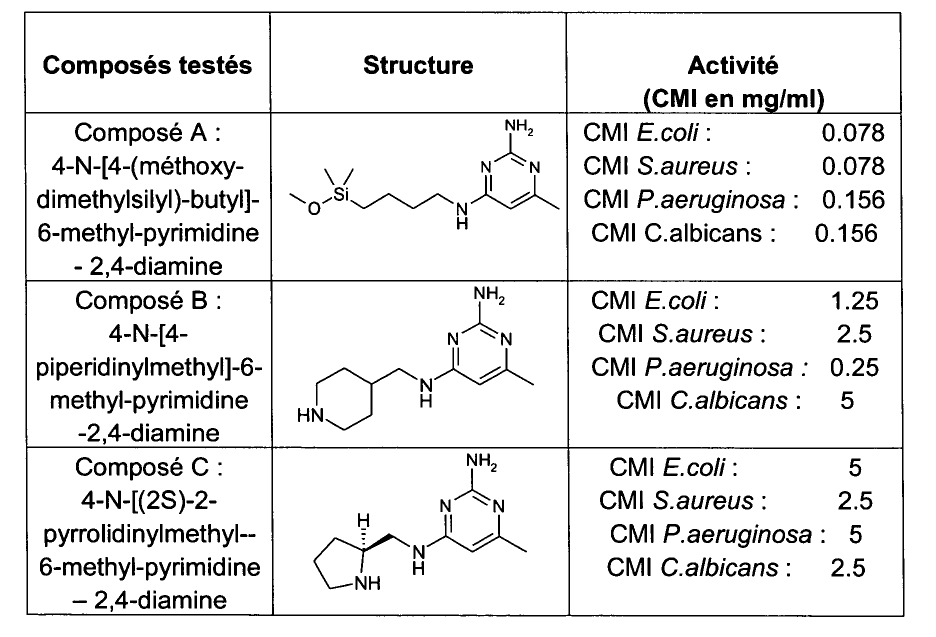

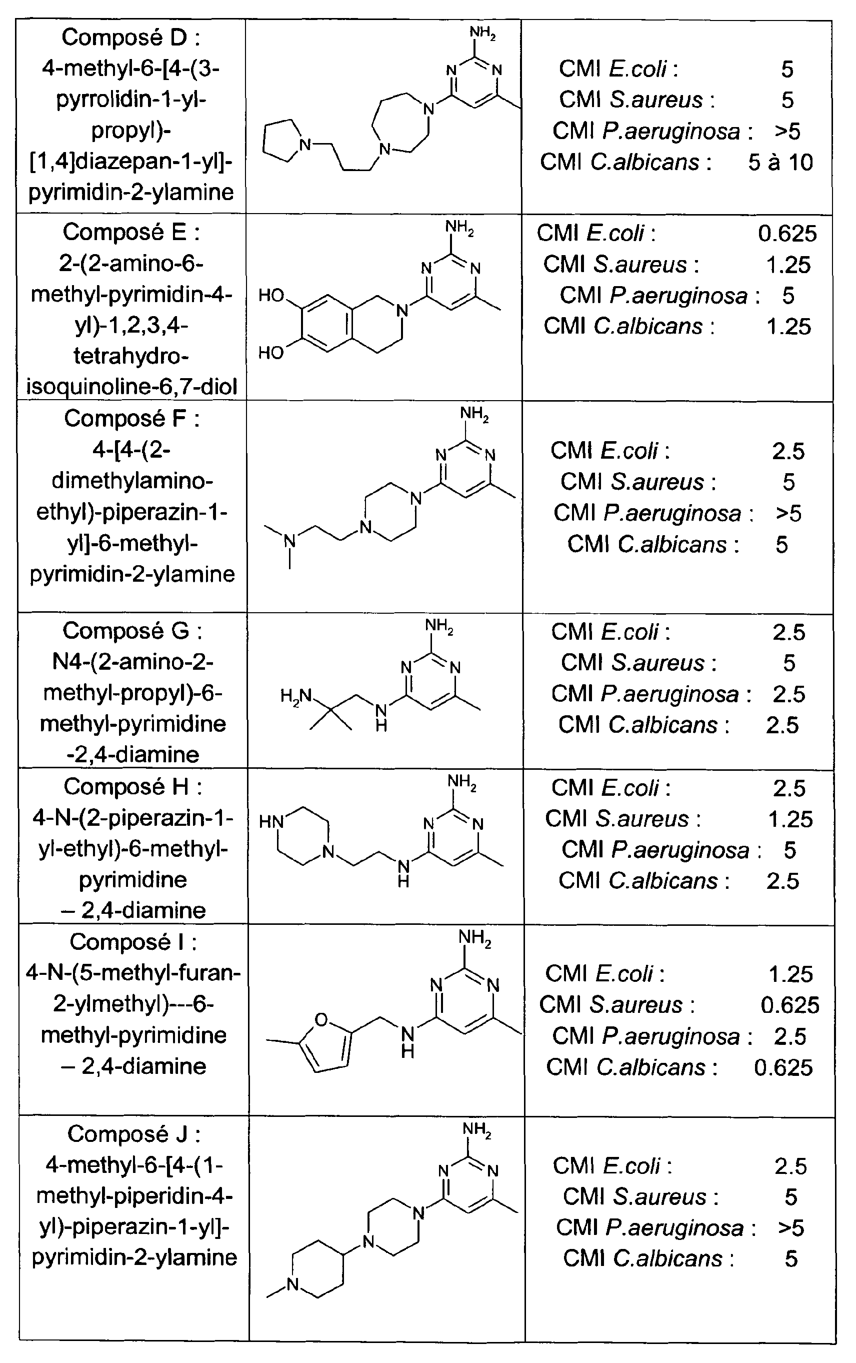

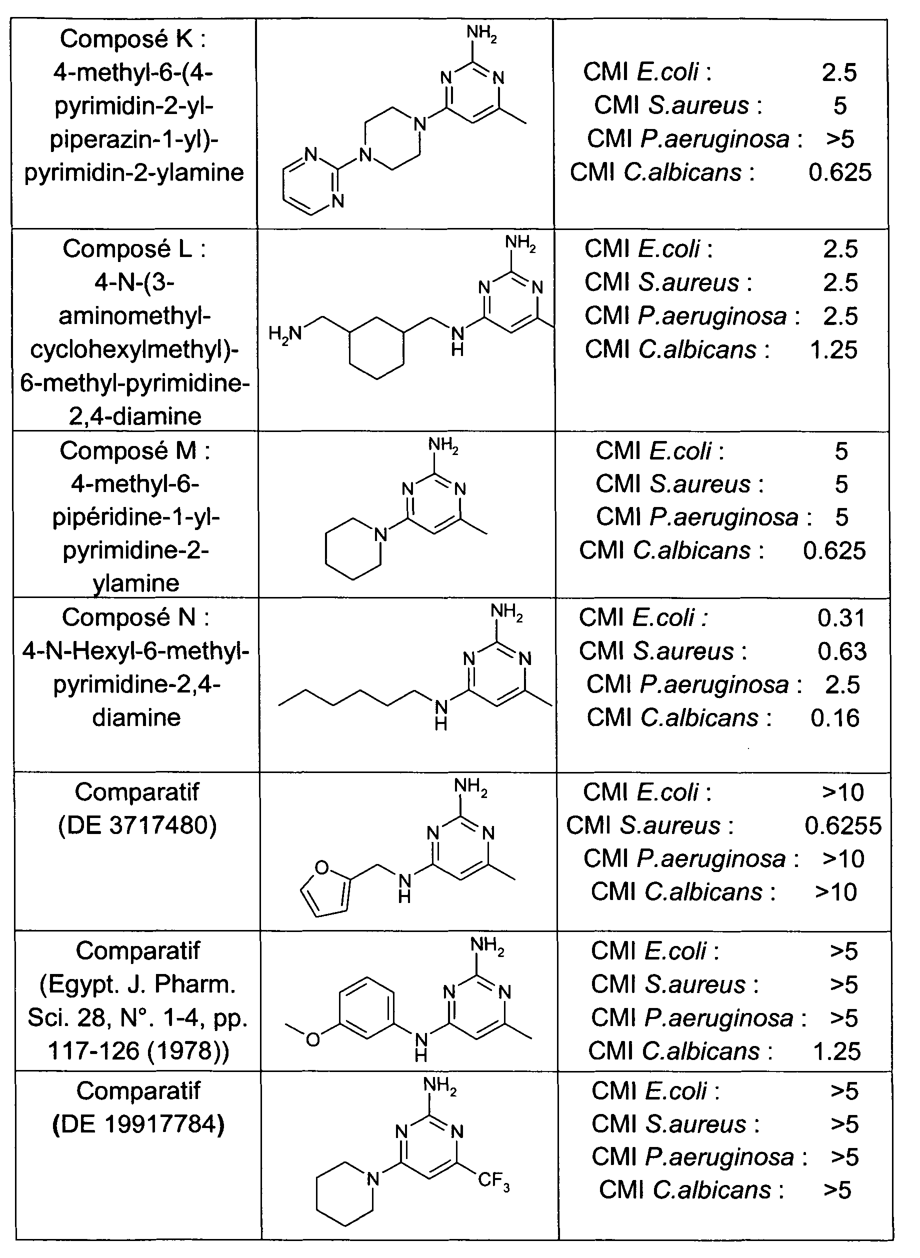

Les résultats montrent que les Concentrations Minimales Inhibitrices (CMI) sont meilleures pour les composés suivant l'invention par rapport aux composés de référence de l'art antérieur.

### EXEMPLE 5

Etude de détermination d'efficacité anti-microbienne des composés de formule (I) dans des supports cosmétiques.

La mesure de la Concentration Minimale Inhibitrice (C.M.I.) développée ci-dessus ne peut être utilisée avec fiabilité pour les moisissures; or les moisissures peuvent également poser de graves problèmes dans la conservation des produits cosmétiques.
En sachant que l'activité d'un composé diminue lorsqu'il est introduit dans une formule et que la méthode de la CMI se fait après dilution du composé dans l'eau, l'efficacité anti-microbienne des composés de formule (I) a été évaluée par la méthode du Challenge Test ou contamination artificielle après avoir introduit lesdits composés dans des supports cosmétiques (lotion, émulsions huile-dans-eau et eau-dans-huile).
La méthode du challenge-test consiste en une contamination artificielle de l'échantillon par des souches microbiennes de collection (bactéries, levures et moisissures) et en une évaluation du nombre de micro-organismes revivifiables sept jours après l'inoculation.
Afin de mettre en évidence l'effet d'un composé de formule (I), l'activité anti-microbienne d'une formule cosmétique contenant 0.25% de 4 methyl-pipéridine-1-yl-pyrimidine-2-ylamine a été comparée avec la même formule seule, après inoculation d'environ 10⁶ germes/gramme de produit cosmétique.

### Lotion :

| | |
|---|---|
| Parfum | 0.01 % |
| Allantoïne | 0.05% |
| Hexylène glycol | 1.00% |
| N-cocoyl amidoéthyl, N-éthoxycarboxymethylglycinate de sodium | 1.10% |
| Eau de bleuet | 1.00% |
| Chlorure de sodium | 0.90% |
| Lauryl éther sulfate de sodium/magnésium (80/20) à 52% dans l'eau | 0.45% |
| 4 methyl-pipéridine-1-yl-pyrimidine-2-ylamine | 0.25% |
| Eau | qsp 100% |

| Souches testées | Nombre d'UFC¹ / gramme de produit après 7 jours de contact germes / produit | |
|---|---|---|
| | Lotion témoin | Lotion + composé |
| *Escherichia coli* | 2.0 10⁶ | <200² |
| *Pseudomonas aeruginosa* | 4.2 10⁶ | <200 |
| *Enterococcus faecalis* | 2.5 10⁴ | 400 |
| *Candida albicans* | 3.9 10⁵ | <200 |
| *Aspergillus niger* | 2.4 10⁵ | 400 |

| | | |
|---|---|---|
| ¹ UFC : Unité Formant Colonie | | |
| ² < 200 UFC : seuil de sensibilité de la méthode. | | |

### Emulsion démaquillante huile dans eau:

| | |
|---|---|
| Hydroxyde de sodium pur | 0.03% |
| Huile de vaseline | 10.00% |
| Palmitate de 2-éthyl hexyle | 10.00% |
| Glycérol | 5.00% |
| Copolymère acide acrylique/acrylate d'alkyle | 0.10% |
| Mélange cétylstéaryl glucoside/alcool cetylstéarylique | 2.45% |
| 4 methyl-pipéridine-1-yl-pyrimidine-2-ylamine | 0.25% |
| Eau | qsp 100% |

| Souches testées | Nombre d'UFC¹ / gramme de produit après 7 jours de contact germes / produit | |
|---|---|---|
| | Emulsion témoin | Emulsion + composé |
| *Escherichia coli* | 2.4 10⁶ | <200² |
| *Pseudomonas aeruginosa* | 3.4 10⁶ | <200 |
| *Enterococcus faecalis* | 7.3 10⁵ | <200 |
| *Candida albicans* | 6.9 10⁵ | <200 |
| *Aspergillus niger* | 2.0 10⁵ | 1.2 10³ |

| | | |
|---|---|---|
| ¹ UFC : Unité Formant Colonie | | |
| ² < 200 UFC : seuil de sensibilité de la méthode. | | |

### Emulsion eau dans huile:

| | |
|---|---|
| Sulfate de magnésium | 0.175% |
| Cyclopentadiméthylsiloxane | 12.000% |
| Glycérol | 3.250% |
| Copolymère acrylamide/acrylamido-2-méthylpropane sulfonique, sel de sodium | 1.000% |
| Mélange distéarate de glycol acétyle /tristéarate de glycéryle | 0.125% |
| Polydiméthyl/méthylcétyl/méthylsiloxane OE | 0.750% |
| 4 methyl-pipéridine-1-yl-pyrimidine-2-ylamine | 0.250% |
| Eau | qsp 100% |

| Souches testées | Nombre d'UFC¹ / gramme de produit après 7 jours de contact germes / produit | |
|---|---|---|
| | Emulsion témoin | Emulsion + composé |
| *Escherichia coli* | 2.9 10⁵ | <200² |
| *Pseudomonas aeruginosa* | 1.0 10⁵ | <200 |
| *Enterococcus faecalis* | 1.2 10⁵ | 8.4 10³ |
| *Candida albicans* | 3.7 10⁵ | <200 |
| *Aspergillus niger* | 5.8 10⁴ | 400 |

| | | |
|---|---|---|
| ¹UFC : Unité Formant Colonie | | |
| ² < 200 UFC : seuil de sensibilité de la méthode. | | |

Cette étude démontre que les composés selon l'invention présentent un spectre antimicrobien très large du fait de leur activité antibactérienne et antifongique. Ce sont donc des conservateurs efficaces, en particulier dans les formulations testées (émulsions huile-dans-eau et eau-dans-huile).

### Exemple 6: Gel pour le visage

- Polyacrylate de glycéryle (Norgel) 30%
- Polyacrylamide/C13-14 Isoparaffine/laureth-7 (Sepigel 305) 2%
- Huile de silicone 10%
- composé de l'exemple N 5%
- Eau qsp 100%

### Exemple 7 : lotion

- composé de l'exemple A 0,2%
- glycérine 2%
- alcool éthylique 20%
- butanol oxyéthyléné (26OE) oxypropyléné (26OP), huile de ricin hydrogénée oxyéthylénée (40 OE) dans l'eau 1%
- eau déminéralisée qsp 100%

### Exemple 8 : Gel traitant

- composé de l'exemple H 1 %
- gomme de xanthane 1 %
- glycérine 2%
- éthanol 20%
- mélange alcool butylique oxyéthyléné (26OE) oxypropyléné (26OP), huile de ricin hydrogénée oxyéthylénée (40OE) dans l'eau 1 %
- parfum qs
- eau déminéralisée qsp 100%

### Exemple 9 : crème nettoyante moussante

- monostéarate d'éthylène glycol 2%
- composé de l'exemple A 0,5%
- silicate de magnésium et d'aluminium hydraté 1,7%
- hydroxypropylméthylcellulose 0,8%
- mélange de cocoyl isethionate de sodium et d'acides gras de coprah (Elfan AT 84 G de Akzo) 15%
- acide stéarique 1,25%
- lauroyl sarcosinate de sodium à 30 % dans l'eau 10%
- parfum qs
- eau déminéralisée qsp 100%

### Exemple 10 : Crème de soin

- tristéarate de sorbitane 1 %
- composé de l'exemple H 1,5%
- homopolymère carboxyvinylique réticulé 0,4%
- gomme de xanthane 0,5%
- copolymère diméthacrylate d'éthylèneglycol/méthacrylate de lauryle 1%
- cyclopentadiméthylsiloxane 6%
- glycérine 3%
- émulsionnant 4%
- parfum qs
- eau déminéralisée qsp 100%

### Exemple 11 : Stick camouflant

- cires (Carnauba et Ozokérite) 14%
- fraction liquide de beurre de karité 4%
- oxydes de titane et de zinc 22%
- oxydes de fer 4%
- composé de l'exemple A 1 %
- polydiméthylsiloxane / silice hydratée 0,1%
- alcool cétylique 1,4%
- isoparaffine qsp 100%

### Exemple 12 : Crème teintée

- lécithine hydrogénée 2,4%
- huile d'amandes d'abricot 6%
- copolymère diméthacrylate d'éthylèneglycol/méthacrylate de lauryle 1%
- stérols de soja oxyéthylénés (5 OE) 1,6%
- composé de l'exemple N 1%
- oxydes de fer 0,9%
- oxyde de titane 5%
- polyacrylamide / C₁₃-C₁₄-Isoparaffine / Laureth-7 (Sepigel 305) 4%
- cyclopentadiméthylsiloxane 6%
- glycérine 6%
- propylène glycol 6%
- parfum qs
- eau déminéralisée qsp 100%

### Exemple 13 : Crème de soin pour le visage (émulsion huile-dans-eau)

| | |
|---|---|
| 4-(2-Amino-6-methyl-pyrimidin-4-ylamino)-butyl-dimethyl-silanol | 0,15% |
| Stéarate de glycérol | 2,00% |
| Polysorbate 60 (Tween 60 vendu par la société ICI) | 1,00% |
| Acide stéarique | 1,40% |
| Acide n-octanoyl-5-salicylique | 0,50% |
| Triéthanolamine | 0,70% |
| Carbomer | 0,40% |
| Fraction liquide du beurre de karité | 12,00% |
| Perhydrosqualène | 12,00% |
| Antioxydant | 0,05% |
| Parfum | 0,5% |
| Eau | qsp 100 % |

### Exemple 14 : Crème nettoyante moussante

- monostéarate d'éthylène glycol 2%
- N4-Hexyl-6-methyl-pyrimidine-2,4-diamine 0,5%
- silicate de magnésium et d'aluminium hydraté 1,7%
- hydroxypropylméthylcellulose 0,8%
- mélange de cocoyl isethionate de sodium et d'acides gras de coprah (Elfan AT 84 G de Akzo) 15%
- acide stéarique 1,25%
- lauroyl sarcosinate de sodium à 30 % dans l'eau 10%
- parfum qs
- eau déminéralisée qsp 100%

## Revendications

1. Composition cosmétique, dermatologique ou pharmaceutique, comprenant, dans un milieu physiologiquement acceptable, au moins un composé de la famille de la 6-méthyl-pyrimidine-2,4-diamine, de formule (I): 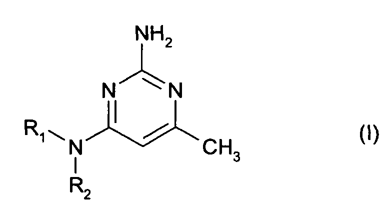 dans laquelle R₁ et R₂ , identiques ou différents, représentent:
- un atome d'hydrogène, étant entendu que R₁ et R₂ ne représentent pas simultanément un atome d'hydrogène,
- un radical hydrocarboné linéaire ou ramifié, saturé ou insaturé, en C1-C20, avantageusement en C1-C6, pouvant être substitué par au moins un radical choisi parmi :
(i) un radical -NH₂, -CN, -CF₃, -OZ ou -COOZ avec Z représentant un atome d'hydrogène ou un radical alkyl en C1-C4 linéaire ou ramifié, saturé ou insaturé, avantageusement un radical méthyle, éthyle, propyle, isopropyle, butyle, isobutyle ou tertiobutyle;
(ii) un radical -NRR' avec R et R' identiques ou différents représentent un radical alkyle en C1-C8, avantageusement en C1-C3, linéaire ou ramifié, saturé ou insaturé; R et R' pris ensemble pouvant former avec l'atome d'azote un hétérocycle, qui peut être aromatique, de 5, 6, 7 ou 8 chaînons pouvant comprendre au moins un hétéro-atome additionnel choisi parmi O, N et/ou S, ledit hétérocycle, y compris l'atome d'azote, pouvant être substitué par une chaîne alkyle en C1-C6, ladite chaîne alkyle pouvant être elle-même substituée par au moins un radical -NR"R"' avec R" et R"' représentant indépendamment l'un de l'autre un atome d'hydrogène ou un radical hydrocarboné, saturé ou insaturé, linéaire ou ramifié, en C1-6 ;
(iii) un radical -Si(CH₃)₂-OH; -Si(CH₃)₂-OEt; -Si(CH₃)₂-O-CH₂-Phényle, -Si(CH₃)₂-OMe ou -Si(iPr)₂-OEt;
(iv) un hétérocycle de 5, 6, 7 ou 8 chaînons comprenant au moins un hétéroatome choisi parmi O, N et/ou S, ledit hétérocycle, y compris l'azote quand il est présent, pouvant être substitué par une chaîne alkyle en C1-C6, avantageusement en C1-C3 ;
(v) un hétérocycle aromatique de 5, 6, 7 ou 8 chaînons, comprenant au moins un hétéroatome choisi parmi O, N et/ou S, ledit hétérocycle pouvant être substitué par une chaîne alkyle en C1-C6; avantageusement méthyle, éthyle ou propyle;
(vi) un cycloalkyle comprenant de 5, 6, 7 ou 8 chaînons, pouvant être substitué par une chaîne alkyle en C1-C6 pouvant elle-même être substituée par au moins un radical -NR"R"' avec R" et R"' représentant indépendamment l'un de l'autre un atome d'hydrogène ou un radical hydrocarboné, saturé ou insaturé, linéaire ou ramifié, en C1-C6; et/ou par un cycloalkyle comprenant de 5, 6, 7 ou 8 chaînons;
- R₁ et R₂ pris ensemble pouvant former avec l'atome d'azote un premier hétérocycle non aromatique de 5, 6, 7 ou 8 chaînons pouvant comprendre au moins un hétéroatome additionnel choisi parmi O, N et/ou S, ledit premier hétérocycle, y compris l'atome d'azote, pouvant être substitué par (a) une chaîne alkyle en C1-C6 pouvant être elle-même substituée par au moins un radical -NR"R"' avec R" et R"' représentant, indépendamment l'un de l'autre, un atome d'hydrogène ou un radical hydrocarboné, saturé ou insaturé, linéaire ou ramifié, en C1-C6; et/ou par (b) un cycloalkyle ou un second hétérocycle, pouvant être aromatique, de 5, 6, 7 ou 8 chaînons, qui peut comprendre au moins un autre hétéroatome choisi parmi O, N et/ou S, ledit cycle ou second hétérocycle pouvant être substitué par une chaîne alkyle en C1-C6;
ainsi que leurs sels.

2. Composition selon la revendication 1, dans laquelle R2 est un radical hydrocarboné linéaire ou ramifié, saturé ou insaturé, en C1-C20, avantageusement en C1-C6, pouvant être substitué par
(i) -NH₂,
(iv) un hétérocycle de 5, 6, 7 ou 8 chaînons comprenant au moins un hétéroatome d'azote, ledit hétérocycle, y compris l'azote, pouvant être substitué par une chaîne alkyle en C1-C6, avantageusement en C1-C3 ;
(v) un hétérocycle aromatique de 5, 6, 7 ou 8 chaînons, comprenant au moins un hétéroatome d'oxygène;
(vi) un cycloalkyle comprenant de 5, 6, 7 ou 8 chaînons, pouvant être substitué par une chaîne alkyle en C1-C6 pouvant elle-même être substituée par au moins un radical -NH2.

3. Composition selon l'une des revendications précédentes, dans laquelle R1 représente l'hydrogène.

4. Composition selon la revendication 1 dans laquelle R₁ et R₂ pris ensemble forment avec l'atome d'azote un premier hétérocycle non aromatique de 5, 6, 7 ou 8 chaînons pouvant comprendre au moins un hétéroatome additionnel d'azote, ledit premier hétérocycle, y compris l'atome d'azote, pouvant être substitué par (a) une chaîne alkyle en C1-C6 pouvant être elle-même substituée par au moins un radical -NR"R"' avec R" et R"' représentant, indépendamment l'un de l'autre, un radical hydrocarboné, saturé ou insaturé, linéaire ou ramifié, en C1-C6; et/ou par (b) un cycloalkyle ou un second hétérocycle, pouvant être aromatique, de 5, 6, 7 ou 8 chaînons, qui peut comprendre au moins un autre hétéroatome choisi parmi O, ledit cycle ou second hétérocycle pouvant être substitué par une chaîne alkyle en C1-C6.

5. Composition selon l'une des revendications précédentes, dans laquelle les sels sont choisis parmi les sels d'acide minéral ou d'acide organique, notamment d'acide minéral choisi parmi les acides chlorhydrique, sulfurique, nitrique et phosphorique, ou d'acide organique choisi parmi les acides carbonique, acétique, succinique, fumarique, lactique, glycolique, citrique, gluconique, salicylique et tartrique.

6. Composition selon l'une des revendications précédentes, dans laquelle le composé est choisi parmi :
- 4-N-[4-(methoxydimethylsilyl)butyl]-6-methyl-pyrimidine-2,4-diamine;
- 4-N-(4-(n-butyl-dimethyl-silanol)) 6-methyl-pyrimidine-2,4-diamine;
- 4-N-[-[3-(ethoxydimethylsilyl)propyl]-6-methyl-pyrimidine-2,4-diamine;
- 4-N-[-[3-(ethoxydiisopropylsilyl)propyl]-6-methyl-pyrimidine-2,4-diamine;
- 4-N-[4-piperidinylmethyl]-6-methyl-pyrimidine-2,4-diamine;
- 4-N-[(2S)-2-pyrrolidinylmethyl]-6-methyl-pyrimidine-2,4-diamine;
- 4-N-[-[1-ethylpyrrolidinyl-2-ylmethyl]-6-methyl-pyrimidine-2,4-diamine;
- 4-methyl-6-[4-(3-pyrrolidin-1-yl-propyl)-[1,4]diazepan-1-yl]-pyrimidin-2-ylamine
- 4-Methyl-6-(4-methyl-[1,4]diazepan-1-yl)-pyrimidin-2-ylamine
- 2-[4-(2-Amino-6-methyl-pyrimidin-4-yl)-[1,4]diazepan-1-yl]-éthanol ;
- 4-(4-Butyl-[1,4]diazepan-1-yl)-6-methyl-pyrimidin-2-ylamine ;
- 4-Methyl-6-(4-phenethyl-[1,4]diazepan-1-yl)-pyrimidin-2-ylamine;
- 4-{4-[2-(4-Fluoro-phenyl)-ethyl]-[1,4]diazepan-1-yl}-6-methyl-pyrimidin-2-ylamine
- 4-Methyl-6-[4-(3-trifluoromethyl-pyridin-2-yl)-[1,4]diazepan-1-yl]-pyrimidin-2-ylamine
- 4-[4-(2-dimethylamino-ethyl)-piperazin-1-yl]-6-methyl-pyrimidin-2-ylamine ;
- 4-Methyl-6-(4-methyl-piperazin-1-yl)-pyrimidin-2-ylamine ;
- 2-[4-(2-Amino-6-methyl-pyrimidin-4-yl)-piperazin-1-yl]-éthanol ;
- 3-[4-(2-Amino-6-methyl-pyrimidin-4-yl)-piperazin-1-yl]-propionitrile ;
- 4-[4-(2-Methoxy-ethyl)-piperazin-1-yl]-6-methyl-pyrimidin-2-ylamine ;
- 4-(4-Cyclopentyl-piperazin-1-yl)-6-methyl-pyrimidin-2-ylamine ;
- 4-[4-(3-Methoxy-propyl)-piperazin-1-yl]-6-methyl-pyrimidin-2-ylamine ;
- 4-Methyl-6-(4-phenyl-piperazin-1-yl)-pyrimidin-2-ylamine ;
- 4-Methyl-6-(4-pyridin-2-yl-piperazin-1-yl)-pyrimidin-2-ylamine ;
- 4-Methyl-6-(4-pyrimidin-2-yl-piperazin-1-yl)-pyrimidin-2-ylamine ;
- 4-Methyl-6-(2,3,5,6-tetrahydro-[1,2']bipyrazinyl-4-yl)-pyrimidin-2-ylamine ;
- 4-(4-Cyclohexyl-piperazin-1-yl)-6-methyl-pyrimidin-2-ylamine ;
- 4-(4-Hexyl-piperazin-1-yl)-6-methyl-pyrimidin-2-ylamine ;
- 4-N-[-(2-amino-2-methyl-propyl)-6-methyl-pyrimidine-2,4-diamine ;
- 4-N-[-(2-piperazin-1-yl-ethyl)-6-methyl-pyrimidine-2,4-diamine ;
- 4-N-[-(5-methyl-furan-2-ylmethyl)-6-methyl-pyrimidine-2,4-diamine ;
- 4-N-[(Furan-2-ylmethyl-)6-methyl-pyrimidine-2,4-diamine ;
- 4-Methyl-6-[4-(1-methyl-piperidin-4-yl)-piperazin-1-yl]-pyrimidin-2-ylamine ;
- 4-Methyl-6-(4-imidazol-2-yl-piperazin-1-yl)-pyrimidin-2-ylamine ;
- 4-N-[-(3-aminomethyl-cyclohexylmethyl)-6-methyl-pyrimidine-2,4-diamine ;
- 4 Methyl-6-pipéridine-1-yl-pirimidine-2-ylamine ;
- 4-Methyl-6-pyrrolidin-1-yl-pyrimidin-2-ylamine ;
- 1-(2-Amino-6-methyl-pyrimidin-4-yl)-pyrrolidin-3-ol ;
- [1-(2-Amino-6-methyl-pyrimidin-4-yl)-pyrrolidin-3-yl]-méthanol ;
- 4-Methyl-6-(2-methyl-pyrrolidin-1-yl)-pyrimidin-2-ylamine ;
- 4-(2-Isopropyl-pyrrolidin-1-yl)-6-methyl-pyrimidin-2-ylamine ;
- 1-(2-Amino-6-methyl-pyrimidin-4-yl)-pyrrolidine-2-carbonitrile ;
- 4-Methyl-6-(3-phenyl-pyrrolidin-1-yl)-pyrimidin-2-ylamine ;
- 4-Methyl-6-(3-pyridin-3-yl-pyrrolidin-1-yl)-pyrimidin-2-ylamine ;
- 4-Methyl-6-(3-pyridin-2-yl-pyrrolidin-1-yl)-pyrimidin-2-ylamine ;
- 4-N-Hexyl-6-methyl-pyrimidine-2,4-diamine.

7. Composition selon l'une des revendications précédentes, dans laquelle le composé de formule (1), seul ou en mélange, est présent en une quantité de 0,001 à 30% en poids, de préférence de 0,01 à 15% en poids, et plus particulièrement de 0,1 à 5% en poids, par rapport au poids total de la composition.

8. Composition selon l'une des revendications précédentes, se présentant sous forme de solution aqueuse, hydroalcoolique, organique ou huileuse; de suspension ou de dispersion dans des solvants ou des corps gras, de type lotion ou sérum; sous forme de dispersion vésiculaire; sous forme d'émulsion E/H, H/E ou multiple telle qu'une crème ou un lait; sous forme de pommade, de gel, de bâtonnet solide, de produits anhydres pâteux ou solides, de mousse notamment aérosol ou de spray.

9. Composition selon l'une des revendications précédentes, comprenant au moins un constituant choisi parmi les corps gras siliconés tels que les huiles, les gommes et les cires de silicone; les huiles et les cires d'origine végétale, minérale, animale et/ou synthétique; les hydrocarbures, les esters et les éthers de synthèse, les alcools gras et les acides gras; de l'eau; un monoalcool en C1-C6; des glycols tels que le propylène glycol, des cétones; un émulsionnant amphotère, anionique, cationique ou non ionique; un épaississant ou un gélifiant hydrophiles ou lipophiles; les additifs hydrophiles ou lipophiles, les actifs notamment cosmétiques, les antioxydants, les parfums, les charges, les pigments, les filtres UV, les absorbeurs d'odeur, les colorants, les hydratants (glycérine), des vitamines, des acides gras essentiels, des polymères liposolubles notamment hydrocarbonés, les opacifiants, les stabilisants, les séquestrants, les conditionneurs, les agents propulseurs.

10. Composition selon l'une des revendications précédentes, comprenant au moins un actif additionnel, notamment choisi parmi, seul ou en mélange: les agents desquamants; les agents hydratants; les agents dépigmentants ou propigmentants; les agents anti-glycation; les inhibiteurs de NO-synthase; les agents stimulant la synthèse de macromolécules dermiques ou épidermiques et/ou empêchant leur dégradation; les agents stimulant la prolifération des fibroblastes et/ou des kératinocytes ou stimulant la différenciation des kératinocytes; les agents myorelaxants; les agents tenseurs; les agents anti-pollution et/ou anti-radicalaire; les agents agissant sur la microcirculation; les agents agissant sur le métabolisme énergétique des cellules.

11. Composition selon l'une des revendications précédentes, comprenant au moins un additif acide et/ou un actif à effet secondaire irritant, de préférence choisi parmi les acides alpha- et bêta-hydroxy-carboxyliques ou alpha et bêta-cétocarboxyliques, et plus particulièrement les alpha- et bêta-hydroxyacides tels que l'acide citrique, l'acide malique, l'acide mandélique, l'acide tartrique, l'acide lactique, l'acide glycolique, les acides de fruits, l'acide salicylique, l'acide n-octanoyl-5-salicylique et l'acide ascorbique.

12. Composition selon l'une des revendications précédentes, se présentant :
- sous la forme d'un produit de maquillage de la peau du visage, du corps ou des lèvres, tel qu'un fond de teint, un fard à joues ou à paupières, un stick anti-cernes, un stick camouflant, un eye-liner, un mascara, un rouge à lèvres, un vernis-à-ongles, un soin des ongles;
- sous la forme d'un produit de soin cosmétique de la peau du visage, du corps y compris le cuir chevelu, des lèvres, tel qu'une base de soin pour les lèvres, une base fixante à appliquer sur un rouge à lèvres classique, une composition solaire ou de bronzage artificiel; une composition de soin (de jour, de nuit, antirides, hydratante, etc.) pour le visage; un gel ou une crème de nettoyage ou de démaquillage;
- sous la forme d'une composition pharmaceutique, notamment dermatologique;
- sous la forme d'une composition solide telle qu'un savon;
- sous la forme d'une composition pour aérosol;
- sous la forme d'une composition capillaire, et notamment un shampooing, une lotion de mise en plis, une lotion traitante, une crème ou un gel coiffant, une composition de teinture (notamment d'oxydation) éventuellement sous forme de shampooing colorant, de lotion restructurante pour cheveux, une composition de permanente,
- sous la forme d'une composition à usage bucco-dentaire, par exemple une pâte dentifrice.

13. Utilisation d'au moins un composé de la famille de la 6-méthyl-pyrimidine-2,4-diamine, de formule (I) telle que définie dans l'une des revendications 1 à 5, comme agent neutralisant basique.

14. Utilisation d'au moins un composé de la famille de la 6-méthyl-pyrimidine-2,4-diamine, de formule (I) telle que définie dans l'une des revendications 1 à 5, comme agent conservateur

15. Utilisation selon la revendication 14, dans laquelle le composé de formule (I) est utilisé comme agent anti-microbien, notamment comme agent anti-bactérien et/ou comme agent anti-fongique.

16. Utilisation selon l'une des revendications 13 à 15, dans une composition cosmétique, dermatologique ou pharmaceutique, comprenant par ailleurs un milieu physiologiquement acceptable.

17. Utilisation selon la revendication 16, dans laquelle le composé de formule (I), seul ou en mélange, est présent en une quantité de 0,001 à 30% en poids, de préférence de 0,01 à 15% en poids, et plus particulièrement de 0,1 à 5% en poids, par rapport au poids total de la composition.

18. Utilisation selon l'une des revendications 13 à 17, dans une composition se présentant sous la forme d'un produit de maquillage de la peau du visage, du corps ou des lèvres; sous la forme d'un produit de soin cosmétique de la peau du visage, du corps y compris le cuir chevelu, des lèvres; sous la forme d'une composition pharmaceutique, notamment dermatologique; sous la forme d'une composition solide telle qu'un savon; sous la forme d'une composition pour aérosol; sous la forme d'une composition capillaire; sous la forme d'une composition à usage bucco-dentaire.

19. Procédé de conservation d'une composition cosmétique, dermatologique ou pharmaceutique, **caractérisé en ce qu'**il consiste à incorporer dans ladite composition au moins un composé de formule (I) tel que défini à l'une des revendications 1 à 5.

20. Composé de la famille de la 6-méthyl-pyrimidine-2,4-diamine, de formule (I): 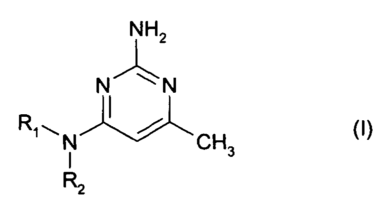 dans laquelle R₁ et R₂ , identiques ou différents, représentent:
- un atome d'hydrogène, étant entendu que R₁ et R₂ ne représentent pas simultanément un atome d'hydrogène,
- un radical hydrocarboné linéaire ou ramifié, saturé ou insaturé, en C1-C20, avantageusement en C1-C6, pouvant être substitué par au moins un radical choisi parmi :
(i) un radical -NH₂, -CN, -CF₃, -OZ ou -COOZ avec Z représentant un atome d'hydrogène ou un radical alkyl en C1-C4 linéaire ou ramifié, saturé ou insaturé, avantageusement un radical méthyle, éthyle, propyle, isopropyle, butyle, isobutyle ou tertiobutyle;
(ii) un radical -NRR' avec R et R' identiques ou différents représentent un radical alkyle en C1-C8, avantageusement en C1-C3, linéaire ou ramifié, saturé ou insaturé; R et R' pris ensemble pouvant former avec l'atome d'azote un hétérocycle, qui peut être aromatique, de 5, 6, 7 ou 8 chaînons pouvant comprendre au moins un hétéro-atome additionnel choisi parmi O, N et/ou S, ledit hétérocycle, y compris l'atome d'azote, pouvant être substitué par une chaîne alkyle en C1-C6, ladite chaîne alkyle pouvant être elle-même substituée par au moins un radical -NR"R"' avec R" et R"' représentant indépendamment l'un de l'autre un atome d'hydrogène ou un radical hydrocarboné, saturé ou insaturé, linéaire ou ramifié, en C1-6 ;
(iii) un radical -Si(CH₃)₂-OH; -Si(CH₃)₂-OEt; -Si(CH₃)₂-O-CH₂-Phényle, -Si(CH₃)₂-OMe ou -Si(iPr)₂-OEt;
(iv) un hétérocycle de 5, 6, 7 ou 8 chaînons comprenant au moins un hétéroatome choisi parmi O, N et/ou S, ledit hétérocycle, y compris l'azote quand il est présent, pouvant être substitué par une chaîne alkyle en C1-C6, avantageusement en C1-C3 ;
(v) un hétérocycle aromatique de 5, 6, 7 ou 8 chaînons, comprenant au moins un hétéroatome choisi parmi O, N et/ou S, ledit hétérocycle pouvant être substitué par une chaîne alkyle en C1-C6; avantageusement méthyle, éthyle ou propyle;
(vi) un cycloalkyle comprenant de 5, 6, 7 ou 8 chaînons, pouvant être substitué par une chaîne alkyle en C1-C6 pouvant elle-même être substituée par au moins un radical -NR"R"' avec R" et R"' représentant indépendamment l'un de l'autre un atome d'hydrogène ou un radical hydrocarboné, saturé ou insaturé, linéaire ou ramifié, en C1-C6; et/ou par un cycloalkyle comprenant de 5, 6, 7 ou 8 chaînons;
- R₁ et R₂ pris ensemble pouvant former avec l'atome d'azote un premier hétérocycle non aromatique de 5, 6, 7 ou 8 chaînons pouvant comprendre au moins un hétéroatome additionnel choisi parmi O, N et/ou S, ledit premier hétérocycle, y compris l'atome d'azote, pouvant être substitué par (a) une chaîne alkyle en C1-C6 pouvant être elle-même substituée par au moins un radical -NR"R"' avec R" et R"' représentant, indépendamment l'un de l'autre, un atome d'hydrogène ou un radical hydrocarboné, saturé ou insaturé, linéaire ou ramifié, en C1-C6; et/ou par (b) un cycloalkyle ou un second hétérocycle, pouvant être aromatique, de 5, 6, 7 ou 8 chaînons, qui peut comprendre au moins un autre hétéroatome choisi parmi O, N et/ou S, ledit cycle ou second hétérocycle pouvant être substitué par une chaîne alkyle en C1-C6;
ainsi que leurs sels.
à l'exception de la 2-amino-4-morpholino-6-méthylpyridine et de la 2-amino-4-pipéridino-6-méthylpyridine.

21. Composé selon la revendication 20, dans lequel R2 est un radical hydrocarboné linéaire ou ramifié, saturé ou insaturé, en C1-C20, avantageusement en C1-C6, de préférence substitué par :
(i) -NH₂,
(iv) un hétérocycle de 5, 6, 7 ou 8 chaînons comprenant au moins un hétéroatome d'azote, ledit hétérocycle, y compris l'azote, pouvant être substitué par une chaîne alkyle en C1-C6, avantageusement en C1-C3 ;
(v) un hétérocycle aromatique de 5, 6, 7 ou 8 chaînons, comprenant au moins un hétéroatome d'oxygène;
(vi) un cycloalkyle comprenant de 5, 6, 7 ou 8 chaînons, pouvant être substitué par une chaîne alkyle en C1-C6 pouvant elle-même être substituée par au moins un radical -NH2.

22. Composé selon l'une des revendications 20 à 21, dans lequel R1 représente l'hydrogène.

23. Composé selon la revendication 20, dans lequel R₁ et R₂ pris ensemble forment avec l'atome d'azote un premier hétérocycle non aromatique de 5, 6, 7 ou 8 chaînons pouvant comprendre au moins un hétéroatome additionnel d'azote, ledit premier hétérocycle, y compris l'atome d'azote, pouvant être substitué par (a) une chaîne alkyle en C1-C6 pouvant être elle-même substituée par au moins un radical -NR"R"' avec R" et R"' représentant, indépendamment l'un de l'autre, un radical hydrocarboné, saturé ou insaturé, linéaire ou ramifié, en C1-C6; et/ou par (b) un cycloalkyle ou un second hétérocycle, pouvant être aromatique, de 5, 6, 7 ou 8 chaînons, qui peut comprendre au moins un autre hétéroatome choisi parmi O, ledit cycle ou second hétérocycle pouvant être substitué par une chaîne alkyle en C1-C6.

24. Procédé de préparation des composés de formule (I) selon l'une des revendications 20 à 23, dans lequel on fait réagir, notamment en une seule étape, la 2-amino-4-chloro-6-methyl-pyrimidine ou de la 2-amino-4-methoxy-6-methyl-pyrimidine avec une base, notamment une amine primaire ou secondaire.

25. Procédé de préparation des composés de formule (1) selon l'une des revendications 20 à 23, **caractérisé en ce qu'**il consiste essentiellement en les étapes suivantes:
(i) dissoudre une amine primaire ou secondaire dans un solvant organique polaire, de préférence choisi parmi l'éthanol, méthanol, THF, dioxanne et N-methyl-pyrrolidone;
(ii) ajouter une base organique ou inorganique, de préférence choisie parmi le carbonate de potassium (K₂CO₃), le carbonate de sodium (Na₂CO₃), le carbonate de calcium (CaCO₃), la soude, la diisopropylethylamine, de préférence la diisopropylethylamine, en une quantité comprise entre 1 et 3 équivalents, préférentiellement entre 1 et 2 équivalents, avantageusement 1,5 à 1,7 équivalents ;
(iii) ajouter la 2-amino-4-chloro-6-methyl-pyrimidine ou la 2-amino-4-methoxy-6-methyl-pyrimidine, de préférence la 2-amino-4-chloro-6-methyl-pyrimidine, en une quantité comprise 0,5 et 1,1 équivalents, avantageusement 0,7 à 0,9 équivalents ;
(iv) laisser le milieu à température ambiante ou le porter à une température comprise entre 20°C et 85°C, de préférence à reflux du solvant, pendant une durée comprise entre 2h et 48h, avantageusement entre 12h et 30h, de préférence 20- 26 h ;
(v) éventuellement évaporer sous vide à sec le milieu réactionnel après retour à température ambiante ;
(vi) éventuellement dissoudre le résidu dans de l'eau et extraire la solution aqueuse avec un solvant organique choisi de préférence parmi l'acétate d'éthyle, le dichlorométhane, le diéthyléther, de préférence l'acétate d'éthyle ;
(vii) éventuellement sécher et évaporer à sec la phase organique puis éventuellement purifier le résidu, notamment par chromatographie sur colonne de gel de silice.
